# EUROPÄISCHE PATENTSCHRIFT

(11) **EP 0 647 231 B1**
(45) Veröffentlichungstag und Bekanntmachung des Hinweises auf die Patenterteilung: **27.08.1997**
(21) Anmeldenummer: 93912910.2
(22) Anmeldetag: 08.06.1993
(51) Int. Cl.: C07D 513/04, A61K 31/33, C07D 495/04, C07D 403/06, C07D 471/04

(54) **N-SUBSTITUIERTE 3-AZABICYCLO[3.2.0]HEPTAN-DERIVATE ALS NEUROLEPTIKA USW.**
N-SUBSTITUTED 3-AZABICYCLO[3.2.0]HEPTANE DERIVATIVES USEFUL AS NEUROLEPTIC AGENTS, ETC.
DERIVES N-SUBSTITUES DE 3-AZABICYCLO[3.2.0]HEPTANE UTILES COMME AGENTS NEUROLEPTIQUES, ETC.

(30) Priorität: 19.06.1992 DE 4219974; 21.12.1992 DE 4243287
(43) Veröffentlichungstag der Anmeldung: 12.04.1995
(73) Patentinhaber: BASF Aktiengesellschaft, 67063 Ludwigshafen (DE)
(72) Erfinder: STEINER, Gerd, D-6719 Kirchheim (DE); UNGER, Liliane, D-6700 Ludwigshafen (DE); BEHL, Berthold, D-6700 Ludwigshafen (DE); TESCHENDORF, Hans-Juergen, D-6724 Dudenhofen (DE); MUNSCHAUER, Rainer, D-6730 Neustadt (DE)
(86) Internationale Anmeldenummer: EP9301438
(87) Internationale Veröffentlichungsnummer: WO9400458

(56) Entgegenhaltungen:
- EP-A- 0 070 053
- EP-A- 0 196 132
- GB-A- 921 507

## Beschreibung

Die Erfindung betrifft neue N-substituierte Azabicycloheptan-Derivate, deren Herstellung und Verwendung zur Herstellung von Pharmawirkstoffen.

Es ist bekannt, daß basisch substituierte 5- oder 6-gliedrige heterocyclische Stickstoff-Derivate Wirkungen als Neuroleptika aufweisen (EP 196 132, EP 70 053, EP 110 435).

Hierbei scheinen die beobachteten hohen Affinitäten zu Serotonin-Rezeptoren neben den Dopamin-Affinitäten eine besondere Rolle zu spielen.

Weiter sind in GB 921 507 3-(Indolylalkyl)-3-azabicyclo[3.2.0]heptan-Derivate beschrieben, die u.a. hypotensive und sedaktive Wirkungen besitzen.

Es wurde nun gefunden, daß N-substituierte 3-Azabicyclo [3.2.0]-heptan-Derivate der Formel I 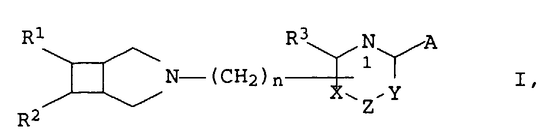 worin
- R¹: eine gegebenenfalls durch Halogenatome, C₁-C₄-Alkyl, Trifluormethyl-, Hydroxy-, C₁-C₄-Alkoxy-, Amino-, Monomethylamino-, Dimethylamino-, Cyano- oder Nitrogruppen mono- oder disubstituierte Phenyl-, Pyridyl-, Thienyl- oder Pyrrolgruppe bedeutet,
- R²: ein Wasserstoffatom oder eine gegebenenfalls durch Halogen, Methoxy, Hydroxy oder Amino substituierte Phenylgruppe ist,
- n: die Zahl O, 1, 2, 3 oder 4 bedeutet,
- R³: ein Wasserstoffatom, eine Hydroxy-, C₁-C₄-Alkyl- oder C₁-C₄-Alkoxygruppe ist, oder zusammen mit dem benachbarten Kohlenstoffatom eine C=O- oder C=S-Gruppe bedeutet,
- X: und Y Kohlenstoffatome, CH-, CH₂-, NH- oder C₁-C₄-Alkyl-N-Gruppen oder Stickstoffatome bedeuten,
- Z: eine direkte Bindung, eine CO-Gruppe, CS-Gruppe oder eine CH- bzw. CH₂-Gruppe, in denen ein Wasserstoffatom gegen eine Hydroxy-, Amino- oder C₁-C₄-Alkoxygruppe oder ein Halogenatom ausgetauscht sein kann, darstellt, und
- A: ein Wasserstoffatom, eine Hydroxy-, Amino-, Mercapto-, C₁-C₄-Alkylamino-, Di-C₁-C₄-alkylamino-, C₁-C₄-Alkylthio- oder C₁-C₄-Alkoxygruppe oder zusammen mit dem benachbarten Kohlenstoffatom eine C=O-Gruppe bedeutet, oder
- A: eine mit Y verknüpfte C₃-C₄-Alkylengruppe bedeutet, die eine oder zwei nicht kumulierte Doppelbindungen enthalten kann und in der eine CH- oder CH₂-Gruppe durch ein Stickstoff- oder Schwefelatom oder eine NH- oder N-CH₃-Gruppe ersetzt sein kann und wobei der Ring entweder durch ein Fluor- oder Chloratom oder eine Methyl-, Methoxy-, Nitro- oder Aminogruppe monosubstituiert oder im Fall eines Benzolrings dieser durch Fluor- oder Chloratome oder Methyl-, Trifluormethyl-, Nitro-, Hydroxy-, Methoxy-, Amino-, Monomethyl- oder Dimethylaminogruppen mono-, di- oder trisubstituiert sein kann,
und worin der Ring im rechten Teil der Formel I am Stickstoffatom Nr. 1 eine C₁-C₄-Alkyl-, Allyl- oder Benzylgruppe tragen und 1 bis 3 nicht kumulierte Doppelbindungen enthalten kann, und deren Salze mit physiologisch verträglichen Säuren wertvolle pharmakologische Eigenschaften besitzen.

Als Substituenten R¹, R², R³ und n sind besonders folgende Bedeutungen zu nennen:
- R¹:: Phenyl, gegebenenfalls durch Fluor, Chlor, Jod, Methoxy, Nitro, Trifluormethyl, Hydroxy oder Amino substituiert,
- R²: Wasserstoff,
- R³ :: Methyl und Hydroxy,
- n:: 2.

Das Ringsystem im rechten Teil der Formel I ist besonders

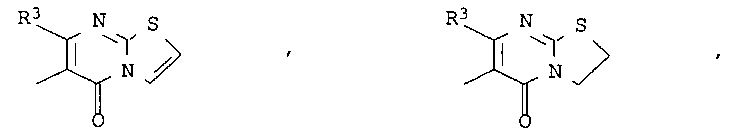

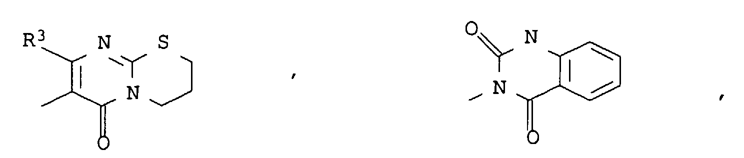

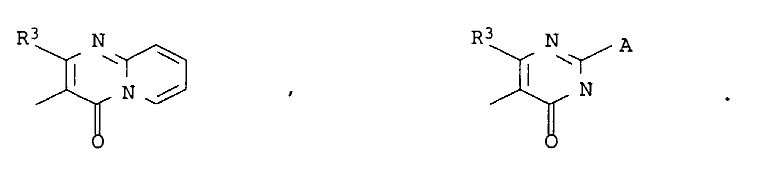

Bevorzugt sind insbesonders die Verbindungen, in denen
- R¹: vorzugsweise in der p-Stellung durch Fluor und Chlor oder in der m-Stellung durch Fluor oder Chlor substituiertes Phenyl
- R²: Wasserstoff und
- R³: Methyl und Hydroxy
sind und sich das Ringsystem im rechten Teil des Moleküls von
7-Methyl-5H-thiazolo[3,2-a]pyrimidin-5-on,
7-Methyl-2,3-dihydro-5H-thiazolo[3,2-a]pyrimidin-5-on,
8-Methyl-3,4-dihydro-2H,6H-pyrimido[2,1-b] [1,3] thiazin-6-on,
2,4-(1H,3H)-Chinazolindion,
2-Methyl-4H-pyrido[1,2-a]pyrimidin-4-on,
6,7,8,9-Tetrahydro-2-methyl-4H-pyrido[1,2-a]pyrimidin-4-on oder
2-Methylamino-3,6-dimethyl-4(3H)pyrimidinon ableitet.

Die folgenden Verbindungen sind als besonders bevorzugt zu nennen:
6-β-[exo-6-p-Fluor-phenyl-3-azabicyclo[3.2.0]heptan-3-yl]-ethyl-7-methyl-5H-thiazolo[3,2-a]pyrimidin-5-on,
6-β-[exo-6-p-Chlor-phenyl-3-azabicyclo[3.2.0]heptan-3-yl]-ethyl-7-methyl-5H-thiazolo[3,2-a]pyrimidin-5-on,
6-β-[exo-6-p-Fluor-phenyl-3-azabicyclo[3.2.0]heptan-3-yl]-ethyl-7-methyl-2,3-dihydro-5H-thiazolo[3,2-a]-pyrimidin-5-on,
7-β-[exo-6-p-Fluor-phenyl-3-azabicyclo[3.2.0]heptan-3-yl]-ethyl-8-methyl-2H,6H-pyrimido[2,1-b] [1,3]thiazin-6-on,
3-β-[exo-6-p-Fluor-phenyl-3-azabicyclo[3.2.0]heptan-3-yl]-ethyl-2,4-(1H,3H)-chinazolindion,
3-β-[exo-6-p-Trifluormethyl-phenyl-3-azabicyclo[3.2.0]-heptan-3-yl]-ethyl-2,4-(1H,3H)-chinazolindion,
3-β-[exo-6-p-Fluor-phenyl-3-azabicyclo[3.2.0]heptan-3-yl]-ethyl-2-methyl-4H-pyrido-[1,2-a]pyrimidin-4-on,
3-β-[exo-6-p-Fluor-phenyl-3-azabicyclo[3.2.0]heptan-3-yl]-ethyl-6,7,8,9-tetrahydro-2-methyl-4H-pyrido[1,2-a]-pyrimidin-4-on,
5-β-[exo-6-p-Fluor-phenyl-3-azabicyclo[3.2.0]heptan-3-yl]-ethyl-2-methylamino-3,6-dimethyl-4(3H)-pyrimidinon.
5-β-[exo-6-m-Chlor-phenyl-3-azabicyclo[3.2.0]heptan-3-yl]-ethyl-2-methylamino-3,6-dimethyl-4(3H)-pyrimidinon
Die erfindungsgemäßen Verbindungen der Formel I lassen sich herstellen, indem man eine Verbindung der Formel II 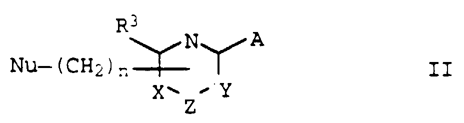 in der n, R³, X, Y, Z und A die oben angegebenen Bedeutungen haben und Nu eine nukleofuge Abgangsgruppe darstellt, mit einem 3-Azabicyclo[3.2.0]heptan-Derivat der Formel III 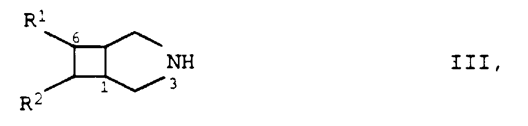 worin R¹ und R² die oben angegebene Bedeutung haben, umsetzt und die so erhaltene Verbindung gegebenenfalls in das Säureadditionssalz einer physiologisch verträglichen Säure überführt.

Als nukleofuge Abgangsgruppe für Nu kommen vorzugsweise Halogenatome, insbesondere Brom oder Chlor, in Betracht.

Die Umsetzung erfolgt zweckmäßig in Gegenwart einer inerten Base, wie Triethylamin oder Kaliumcarbonat, als säurebindendes Mittel in einem inerten Lösungsmittel, wie einem cyclischen gesättigten Ether, insbesondere Tetrahydrofuran oder Dioxan, oder einem Benzolkohlenwasserstoff, wie Toluol oder Xylol.

Die Umsetzung erfolgt in der Regel bei Temperaturen von 20 bis 150°C, insbesondere von 80 bis 140°C, und ist im allgemeinen innerhalb von 1 bis 10 Stunden beendet.

Die erfindungsgemäßen Verbindungen der Formel I können entweder durch Umkristallisation aus den üblichen organischen Lösungsmitteln, bevorzugt aus einem niederen Alkohol, wie Ethanol, umkristallisiert oder durch Säulenchromatographie gereinigt werden.

Racemate lassen sich in einfacher Weise durch klassische Spaltung mit optisch aktiven Carbonsäuren, z.B. Weinsäure-Derivaten, in einem inerten Lösungsmittel, z.B. niederen Alkoholen, in die Enantiomeren auftrennen.

Die freien 3-Azabicyclo[3.2.0]heptan-Derivate der Formel I können in üblicher Weise in das Säureadditionssalz einer pharmakologisch verträglichen Säure überführt werden, vorzugsweise durch Versetzen einer Lösung mit einem Äquivalent der entsprechenden Säure. Pharmazeutisch verträgliche Säuren sind beispielsweise Salzsäure, Phosphorsäure, Schwefelsäure, Methansulfonsäure, Amidosulfonsäure, Maleinsäure, Fumarsäure, Oxalsäure, Weinsäure oder Zitronensäure.

Die erfindungsgemäßen Verbindungen weisen wertvolle pharmakologische Eigenschaften auf. Sie können als Neuroleptika (insbesondere atypische), Antidepressiva, Sedativa, Hypnotika, ZNS-Protektiva oder Muskelrelaxantien Verwendung finden. Mehrere der genannten Wirkungsqualitäten können kombiniert bei einer erfindungsgemäßen Verbindung auftreten. Der pharmakologische Wirkungsnachweis erfolgt sowohl in vivo wie auch in vitro, wobei die Substanzcharakterisierung insbesondere durch die teilweise sehr hohe und selektive Affinität zu Rezeptor-Subtypen, z.B. Dopamin D₁-, D₂-, D₃-, D₄-Rezeptoren; Serotonin 1A-, 1D- und 2-Rezeptoren, Alpha 1- und 2-Rezeptoren; Histamin 1- sowie Muscarin-Rezeptoren, möglich ist.

Für die in vivo Charakterisierung wurden folgende Methoden herangezogen:
a) Beeinflussung der Orientierungsmotilität
   Mäuse zeigen in einer neuen Umgebung ein vermehrtes Explorationsverhalten, das sich in einer gesteigerten motorischen Aktivität äußert. Diese motorische Aktivität wird in Lichtschrankenkäfigen für die Zeit von 0 - 30 min nach Einsetzen der Tiere (NMRI-Mäuse, weibl.) in die Käfige gemessen. ED50: Dosis, die die motorische Aktivität im Vergleich zu Placebo-behandelten Kontrollen um 50 % reduziert.
b) Apomorphin-Antagonismus
   Weibliche NMRI-Mäuse erhalten 1,21 mg/kg Apomorphin s.c. Apomorphin führt in dieser Dosis zu einer motorischen Aktivierung, die sich, wenn man die Tiere in Maschendrahtkäfigen hält, in einem permanenten Klettern äußert. Das Klettern wird mit einem Score bewertet (alle 2 min während 30 min):
   0: Tier hat vier Pfoten am Boden
   1: Tier hat zwei Pfoten am Draht
   2: Tier hat vier Pfoten am Draht (klettert).
   Durch Vorbehandlung mit Antipsychotika ist das Kletterverhalten zu hemmen.
   ED50: Dosis, die die Kletteraktivität der Tiere im Vergleich zu Placebo-behandelten Kontrollen um 50 % hemmt.
c) Methamphetamin-Antagonismus
   Weibliche NMRI-Mäuse erhalten 1 mg/kg Methamphetamin p.o. und werden nach 30 min in Lichtschrankenkäfige zur Messung der motorischen Aktivität eingesetzt (2 Tiere/Käfig, 4 Käfige/ Dosis). Die Prüfsubstanzen werden 30 min vor Methamphetamin oral gegeben. Die Aktivitätssteigerung durch Methamphetamin wird für die Zeit 15 bis 60 min nach Einsetzen der Tiere in die Meßkäfige als Differenz von Methamphetaminkontrollen zu Placebokontrollen berechnet und gleich 100 % gesetzt. Die ED100 ist die Dosis der Prüfsubstanz, die die Aktivitätssteigerung vollständig aufhebt.
d) L-5-HTP-Antagonismus
   Weibliche Sprague-Dawley-Ratten erhalten L-5-HTP in einer Dosis von 316 mg/kg i.p. Die Tiere entwickeln darauf ein Erregungssyndrom, von dem Symptome
   - for paw treading und
   - tremor
   mit Hilfe eines Scores (O = nicht vorhanden, 1 = mäßig, 2 = deutlich ausgeprägt) alle 10 min in der Zeit von 20 bis 60 min nach L-5-HTP-Gabe bewertet werden. Im Mittel wird nach L-5-HTP-Gabe ein Score von 17 erreicht. Die Prüfsubstanzen p.o. werden 60 min vor L-5-HTP gegeben. Als ED50 wird die Dosis errechnet, die im Mittel den Kontrollscore um 50 % vermindert.

Die aufgeführten Methoden sind geeignet, Substanzen als Antipsychotika zu charakterisieren; insbesondere die Hemmung von durch Methamphetamin induzierter motorischer Stimulierung gilt als prädiktiv für eine antipsychotische Wirkung. Mit der Hemmung des L-5-HTP-Syndroms kann eine Serotonin-antagonistische Wirkung aufgezeigt werden, eine Wirkungsqualität, wie sie für die sogenannten atypischen Neuroleptika charakteristisch ist.

In diesen Tests zeigen die neuen Verbindungen eine gute Wirkung.

Die Erfindung betrifft dementsprechend auch ein therapeutisches Mittel, gekennzeichnet durch einen Gehalt an einer Verbindung der Formel I oder deren pharmakologisch verträgliches Säureadditionssalz als Wirkstoff neben üblichen Träger- und Verdünnungsmitteln, sowie die Verwendung der neuen Verbindungen bei der Bekämpfung von Krankheiten.

Die erfindungsgemäßen Verbindungen können in üblicher Weise oral oder parenteral, intravenös oder intramuskulär verabfolgt werden.

Die Dosierung hängt vom Alter, Zustand und Gewicht des Patienten sowie von der Applikationsart ab. In der Regel beträgt die tägliche Wirkstoffdosis zwischen etwa 1 und 100 mg/kg Körpergewicht bei oraler Gabe und zwischen 0,1 und 10 mg/kg Körpergewicht bei parenteraler Gabe.

Die neuen Verbindungen können in gebräuchlichen galenischen Applikationsformen fest oder flüssig angewendet werden, z.B. als Tabletten, Filmtabletten, Kapseln, Pulver, Granulate, Dragees, Suppositorien, Lösungen, Salben, Cremes oder Sprays. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et. al: Pharmazeutische Technologie, Thieme-Verlag, Stuttgart, 1978). Die so erhaltenen Applikationsformen enthalten den Wirkstoff normalerweise in einer Menge von 1 bis 99 Gew.-%.

Die als Ausgangsstoffe für die Synthese der neuen Verbindungen benötigten Substanzen der Formel II sind bekannt oder lassen sich gemäß den in der Literatur beschriebenen Herstellmethoden aus analogen Ausgangsmaterialien synthetisieren.

Die Substanzen der Formel III lassen sich herstellen, indem man ein Amin der Formel 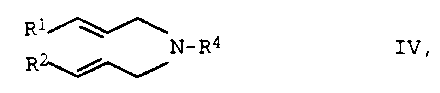 worin R¹ und R² die oben angegebenen Bedeutungen besitzen und R⁴ Wasserstoff, Acetyl, Benzyl oder Trifluoracetyl bedeutet, photochemisch einer 2+2 Cycloaddition unterwirft und gegebenenfalls eine Acyl- oder Benzylgruppe abspaltet.

Die Photoreaktion gelingt gut in einem inerten Lösungsmittel, vorzugsweise Aceton, bei Temperaturen von 20 bis 80°C. Als Lichtquelle eignet sich besonders gut eine Quecksilberhochdrucklampe. Es ist gegebenenfalls vorteilhaft, die Photocycloaddition in einer Quarzapparatur unter einer Stickstoffatmosphäre gegebenenfalls unter Zusatz von etwa 1 Mol Salzsäure pro Mol Amin durchzuführen.

Die Photocycloaddition verläuft in den meisten Fällen hochdiastereoselektiv zu den bicyclischen Verbindungen III mit der exo-Konfiguration bezüglich R¹ und R²: 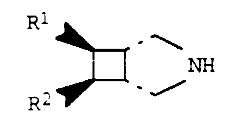 Durch Racematspaltung, z.B. mit optisch aktiven Weinsäure-Derivaten, lassen sich die beiden Enantiomeren rein isolieren.

Die Abspaltung einer Acylgruppe gelingt nach bekannten Methoden. Analoges gilt für die Entfernung einer Benzylgruppe.

Die Amine der Formel IV sind literaturbekannt oder lassen sich herstellen, indem man entweder einen Aldehyd R¹-CHO mit Vinylmagnesiumchlorid zum Allylalkohol V 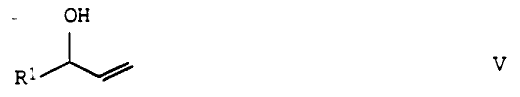 umsetzt, anschließend mit Chlorwasserstoff zum Allylchlorid VI 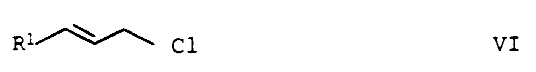 umlagert und zuletzt mit dem entsprechenden Allylamin VII 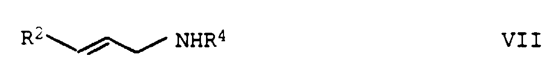 substituiert oder man unterzieht einen Zimtaldehyd VIII 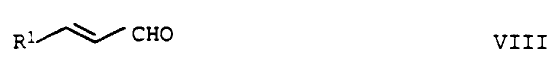 direkt der reduktiven Aminierung mit dem Allylamin VII mit R⁴ gleich Wasserstoff.

Die folgenden Beispiele dienen zur Erläuterung der Erfindung:
A Herstellung der Ausgangsmaterialien
   aa) 1-(4-Fluor-phenyl)-allylalkohol
      In einen 4-1-Rührkolben wurden unter Stickstoff 1550 ml (2,0 M) einer 1,29 M Lösung von Vinylmagnesiumchlorid in Tetrahydrofuran gefüllt. Anschließend wurde unter Rühren und unter Stickstoff innerhalb von 30 min bei 30-35°C eine Lösung von 222,0 g (1,764 M) 4-Fluor-benzaldehyd in Tetrahydrofuran zugegeben, wobei der Reaktionsansatz mit Eis gekühlt wurde. Das Reaktionsgemisch wurde noch 2,5 h unter Stickstoff bei Raumtemperatur gerührt. Danach wurde unter Rühren und Kühlen mit Eis 180 ml Wasser zugesetzt, abgesaugt und der Filterrückstand dreimal mit 150 ml Tetrahydrofuran gewaschen. Die Filtrate wurden vereinigt, mit Natriumsulfat getrocknet und eingeengt. Man erhielt 265,7 g (99 %) Produkt in Form eines gelbbraunen Öls.
   ab) 3-(4-Fluor-phenyl)-allylchlorid
      273,6 g (1,798 M) l-(4-Fluor-phenyl)-allylalkohol wurden unter Rühren in 2000 ml Methanol gelöst. Anschließend wurden in 3 h 101,0 g (2,770 M) Chlorwasserstoff eingeleitet, wobei die Temperatur auf bis zu 37°C stieg. Es wurde 1 h nachgerührt. Nach dem Waschen mit 600 ml eiskaltem Wasser und einer Mischung aus 150 ml gesättigter Kochsalzlösung und 150 ml Wasser wurde die organische Phase über Natriumsulfat getrocknet und eingeengt. Man erhielt 294,6 g (98 %) eines braunen Öls.
   ac) N-Allyl-N-[3-(4-fluor-phenyl)-allyl]-amin
      Zu einer Lösung von 795,0 g (13,92 M) Allylamin in 360 ml Toluol wurden unter Rückfluß innerhalb von 25 min 231,8 g (1,359 M) 3-(4-Fluor-phenyl)-allylchlorid zugegeben und noch 1 h bei Rückflußtemperatur nachgerührt. Anschließend wurde über eine 10 cm-Destillationskolonne (5 mm Glasringe) bei einer Badtemperatur von bis zu 125°C 1000 ml abdestilliert. Der Destillationsrückstand wurde mit 1000 ml Wasser versetzt und mit 38 %iger Salzsäure auf pH 0,7 eingestellt. Die organische Phase wurde abgetrennt und verworfen. Die wäßrige Phase wurde mit 50 %iger Natronlauge auf pH 12,7 eingestellt und mit Toluol extrahiert und eingeengt. Der Rückstand wurde über eine Kolonne bei 0,7 bis 1 mbar destilliert. Bei einer Badtemperatur von 120-160°C erhielt man 191,8 g (74 %) eines hellgelben Öls.
   ad) exo-6-(p-Fluor-phenyl)-3-azabicyclo[3.2.0]heptan
      19,4 g (102 mM) N-Allyl-N-[3-(4-fluor-phenyl)-allyl]-amin in 130 ml Aceton wurden mit 130 ml 10 %iger Salzsäure sowie mit 600 mg Michlers Keton versetzt und unter Stickstoff 55 h mit einer 150 Watt Quecksilber-Hochdrucklampe in einer Quarzapparatur bei Raumtemperatur bestrahlt. Anschließend engte man den Reaktionsansatz ein und verteilte den Rückstand zwischen Methylenchlorid und Wasser. Man stellte mit wäßriger Ammoniaklösung alkalisch und extrahierte die wäßrige Phase noch zweimal mit Methylenchlorid nach. Die vereinigten organischen Phasen wurden mit Natriumsulfat getrocknet und eingeengt.
      Ausbeute 19,3 g (99 %), Schmp. 165-166°C (Maleinat).
      Zur Trennung der Antipoden versetzte man 15,0 g (78,5 mM) des Racemats zu einer Lösung von 31,7 g (78,5 mM) (-)-Di-O-toluoyl-L-weinsäure in 300 ml siedendem Ethanol. Die beim Abkühlen unter Rühren ausfallenden Kristalle (13,8 g) wurden unter Nachwaschen mit Ethanol abgesaugt und aus 200 ml Ethanol unter Zusatz von 200 ml Wasser umkristallisiert. Freisetzen der Base lieferte den (+)-Antipoden (5,5 g) mit [α]_{D} = + 97,0° (EtOH, c = 0,969).
      Aus der obigen Mutterlauge kristallisierten über Nacht 14,2 g des Salzes, das aus 400 ml Ethanol (Abfiltrieren des unlöslichen Anteils in der Siedehitze) umkristallisiert wurde (Einengen auf 300 ml). Freisetzen der Base ergab 4,0 g des -(-)-Antipoden mit [α]_{D} = - 96,0° (EtOH, c = 0,940).
      Die exo-Phenyl-Konfigurationen wurden mittels Röntgenstrukturanalyse nachgewiesen.
   ae) exo-6-Phenyl-3-azabicyclo[3.2.0]heptan
      50,0 g (28,9 mM) N-Cinnamyl-N-allylamin in 1600 ml Aceton wurden mit 300 ml 10 %iger Salzsäure versetzt und unter Stickstoff 48 h mit einer 150 Watt Quecksilber-Hochdrucklampe in einer Quarzapparatur bei Raumtemperatur bestrahlt. Anschließend engte man den Reaktionsansatz ein und verteilte den Rückstand zwischen Methylenchlorid und Wasser. Man stellte mit wäßriger Ammoniaklösung alkalisch und extrahierte die wäßrige Phase noch zweimal mit Methylenchlorid nach. Die vereinigten organischen Phasen wurden mit Natriumsulfat getrocknet und eingeengt. Ausbeute 49,0 g (98 %) viskoses Öl,
      Schmp. 177 bis 178°C (Maleinat).
   af) exo-6,7-Diphenyl-3-azabicyclo[3.2.0]heptan
      Zu 12,0 g (35,4 mM) exo-6,7-Diphenyl-3-benzyl-3-azabicyclo[3.2.0]heptan in einer Mischung aus 300 ml n-Propanol und 16 ml Wasser wurden 16,0 g (254 mM) Ammoniumformiat sowie 2,0 g Palladium (10 %ig) auf Kohle gegeben und die Reaktionsmischung 4 h am Rückfluß gekocht (Entwicklung von Kohlendioxid). Nach dem Abkühlen saugte man vom Katalysator ab, wusch mit Propanol und Methylenchlorid nach und engte das Filtrat ein. Man verteilte den Rückstand zwischen Methylenchlorid und Wasser, stellte mit wäßriger Ammoniaklösung alkalisch und extrahierte die wäßrige Phase noch zweimal mit Methylenchlorid nach. Die vereinigten organischen Phasen wurden mit Natriumsulfat getrocknet und eingeengt. Man erhielt 8,1 g (92 %) Produkt, Schmp. 140 bis 142°C (Maleinat).
   ag) N-Allyl-N-3-(3,5-dichlorphenyl)-allylamin
      Zu 12,0 g (59,7 mM) 3,5-Dichlorzimtaldehyd in 180 ml Methylenchlorid wurden 4,5 ml (60 mM, 3,4 g) Allylamin sowie 17,0 g Natriumsulfat gegeben und die Reaktionsmischung 24 h bei Raumtemperatur gerührt. Danach wurde das Natriumsulfat abfiltriert, mit Methylenchlorid nachgewaschen und das Filtrat zur Trochene eingeengt. Das so erhaltene gelbe Öl wurde in 200 ml absolutem Methanol gelöst, und unter Stickstoff wurden portionsweise 2,5 g (66,0 mM) Natriumborhydrid zugegeben. Die sich leicht erwärmende Reaktionsmischung wurde noch 1 h nachgerührt und danach mit 10 %iger Salzsäure neutralisiert (pH = 7). Das Lösungsmittel wurde im Vakuum entfernt und der verbleibende Rückstand in Methylenchlorid aufgenommen. Die organische Phase wurde zweimal mit Wasser gewaschen, über Natriumsulfat getrocknet und eingeengt. Der verbleibende Rückstand wurde mittels Säulenchromatographie (Kieselgel, Methylenchlorid + 5 % Methanol) gereinigt.
      Ausbeute: 9,2 g (63 %) gelbes Öl.
   ah) N-Allyl-2,2,2-trifluoro-N-[3-(3-pyridyl)-allyl]-acetamid
      Zu einer Lösung von 10,0 g (57,5 mM) N-Allyl-N-3-(3-pyridyl)-allylamin und 10,7 ml Triethylamin in 100 ml Tetrahydrofuran wurden bei 0°C 16,1 g (76,6 mM) Trifluoracetanhydrid langsam zugetropft. Es wurde noch 2 h bei Raumtemperatur nachgerührt. Danach wurde die Reaktionslösung auf 250 ml Eiswasser gegossen und dreimal mit je 150 ml Methyl-tert.-butylether extrahiert. Die vereinigten organischen Phasen wurden über Natriumsulfat getrocknet und eingeengt. Ausbeute: 14,3 g (92 %) dunkelbraunes Öl.
   ai) 2,2,2-Trifluoro-1-[exo-6-(3-pyridyl)-3-azabicyclo[3.2.0]-hept-3-yl]-ethanon
      14,0 g (51,8 mM) N-Allyl-2,2,2-trifluoro-N-[3-(3-pyridyl)-allyl]-acetamid wurden in 140 ml Aceton gelöst, mit 30 ml 10 %iger wäßriger Salzsäure versetzt und unter Stickstoff 48 h mit einer 150 Watt Quecksilber-Hochdrucklampe in einer Duranglasapparatur bei Raumtemperatur bestrahlt. Danach wurde die Reaktionslösung eingeengt, in 150 ml Wasser aufgenommen und mit wäßriger Ammoniaklösung auf pH 8-9 eingestellt. Die wäßrige Phase wurde zweimal mit tert.-Butyl-methylether extrahiert, die vereinigten organischen Phasen über Natriumsulfat getrocknet und eingeengt. Der verbleibende Rückstand wurde mittels Säulenchromatographie (Kieselgel, Methylenchlorid + 2 % Methanol) fraktioniert. Man erhielt 6,2 g (42 %) unverändertes N-Allyl-2,2,2-trifluoro-N-[3-(3-pyridyl)-allyl]-acetamid und 3,7 g (26 %) 2,2,2-Trifluoro-1-[exo-6-(3-pyridyl)-3-azabicyclo[3.2.0]hept-3-yl]-ethanon als dunkles Öl.
   ak) exo-6-(3-Pyridyl)-3-azabicyclo[3.2.0]heptan
      Zur Lösung von 3,7 g (13,7 mM) 2,2,2-Trifluoro-1-[exo-6-(3-pyridyl)-3-azabicyclo[3.2.0]hept-3-yl]-ethanon in 50 ml Ethanol wurden 2,5 g Kaliumhydroxid-Plätzchen gegeben. Die Reaktionslösung wurde noch 2 h bei Raumtemperatur nachgerührt und anschließend auf 100 ml Eiswasser gegossen. Die wäßrige Phase wurde dreimal mit tert.-Butyl-methylether extrahiert, die vereinigten organischen Phasen wurden über Natriumsulfat getrocknet und eingeengt. Ausbeute 2,3 g (96 %) gelbes Öl,
      Schmp. 202-205°C (Hydrochlorid).

Analog lassen sich folgende Substanzen herstellen:
al) exo-6-(m-Fluor-phenyl)-3-azabicyclo[3.2.0]heptan
am) exo-6-(o-Fluor-phenyl)-3-azabicyclo[3.2.0]heptan, Schmp. 118-120°C (Maleinat)
an) exo-6-(p-Chlor-phenyl)-3-azabicyclo[3.2.0]heptan, Schmp. 152-154°C (Maleinat)
ao) exo-6-(m-Chlor-phenyl)-3-azabicyclo[3.2.0]heptan, Schmp. 130-132°C (Maleinat)
ap) exo-6-(p-Methoxy-phenyl)-3-azabicyclo[3.2.0]heptan
aq) exo-6-(m-Methoxy-phenyll-3-azabicyclo[3.2.0]heptan
ar) exo-6-(p-Nitro-phenyl)-3-azabicyclo[3.2.0]heptan, Schmp, 158-160°C (Maleinat)
as) exo-6-(m-Nitro-phenyl)-3-azabicyclo[3.2.0]heptan
at) exo-6-(p-Trifluormethyl-phenyl)-3-azabicyclo[3.2.0]heptan, Schmp. 155-156°C (Maleinat)
au) exo-6-(m-Trifluormethyl-phenyl)-3-azabicyclo[3.2.0]heptan
av) exo-6-(3,4-Dichlor-phenyl)-3-azabicyclo[3.2.0]heptan
aw) exo-6-(3,5-Dichlor-phenyl)-3-azabicyclo[3.2.0]heptan, Schmp. >250°C (Hydrochlorid)
ax) exo-6-(3,4-Dimethoxy-phenyl))-3-azabicyclo[3.2.0]heptan
ay) exo-6-(m-Hydroxy-phenyl)-3-azabicyclo[3.2.0]heptan
az) exo-6-(p-Hydroxy-phenyl)-3-azabicyclo[3.2.0]heptan
ba) exo-6-(3,4-Dihydroxy-phenyl)-3-azabicyclo[3.2.0]heptan
bb) exo-6-(p-Methyl-phenyl)-3-azabicyclo[3.2.0]heptan
bc) exo-6-(m-Methyl-phenyl)-3-azabicyclo[3.2.0]heptan
bd) exo-6-(p-t-Butyl-phenyl)-3-azabicyclo[3.2.0]heptan, Schmp. >255°C (Hydrochlorid)
be) exo-6-(m-Amino-phenyl)-3-azabicyclo[3.2.0]heptan
bf) exo-6-(p-Amino-phenyl)-3-azabicyclo[3.2.0]heptan
bg) exo-6-(p-Cyano-phenyl)-3-azabicyclo[3.2.0]heptan, Schmp. 168-178°C (Maleinat)
bh) exo-6-Thien-2-yl-3-azabicyclo[3.2.0]heptan, Schmp. 180-182°C (Hydrochlorid)
bi) exo-6-Thien-3-yl-3-azabicyclo[3.2.0]heptan, Schmp. 143-145°C (Hydrochlorid)
bk) exo-6-(5-Chlor-thien-2-yl)-3-azabicyclo[3.2.0]heptan, Schmp. 156-157°C (Maleinat)
bl) exo-6-Pyrrol-2-yl-3-azabicyclo[3.2.0]heptan
bm) exo-6-Pyrid-4-yl-3-azabicyclo[3.2.01heptan
bn) exo-6-Pyrid-2-yl-3-azabicyclo[3.2.0]heptan

### B Herstellung der Endprodukte

### Beispiel 1

6-β-[exo-6-p-Fluor-phenyl-3-azabicyclo[3.2.0]heptan-3-yl]-ethyl-7-methyl-5H-thiazolo[3,2-a]pyrimidin-5-on Dihydrochlorid 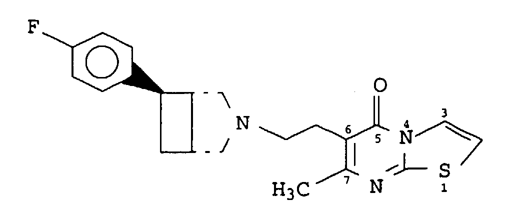 2,5 g (13,1 mM) exo-6-p-Fluor-phenyl-3-azabicyclo[3.2.0]heptan in 40 ml Xylol wurden mit 3,6 g (26 mM) 6-(2-Chlor-ethyl) -7-methyl-5H-thiazolo[3,2-a]pyrimidin-5-on sowie mit 5,6 g (40 mM) fein pulverisiertem Kaliumcarbonat und 0,5 g Kaliumjodid versetzt und unter gutem Rühren 11 h unter Rückfluß gekocht.

Nach dem Abkühlen engte man am Rotationsverdampfer ein und verteilte den Rückstand zwischen Methylenchlorid und Wasser.

Die wäßrige Phase wurde zweimal mit Methylenchlorid nachextrahiert und darauf die organische Phase nach dem Trocknen mit Natriumsulfat eingeengt. Das Rohprodukt (7,7 g) reinigte man durch Säulenchromatographie (Kieselgel, Laufmittel Methylenchlorid/Methanol 96/4).

Die freie Base (3,5 g) nahm man in 150 ml Ether auf, filtrierte die unlöslichen Flocken ab und versetzte die Etherlösung mit überschüssiger etherischer Salzsäure. Anschließend saugte man die Festkörper unter Stickstoff in der Kälte ab, wusch das Hydrochlorid mit reichlich Ether nach und trocknete das Salz auf der Nutsche unter Stickstoff. Man isolierte 3,5 g (60 %) Produkt x 2 HCl, Schmp. 222 bis 224°C. Das Maleinat schmilzt bei 133 bis 135°C.

Analog lassen sich herstellen:
1a. (+)-6-β-[exo-6-p-Fluor-phenyl-3-azabicyclo[3.2.0]heptan-3-yl]-ethyl-7-methyl-5H-thiazolo[3,2-a]pyrimidin-5-on Maleinat, Schmp. 158-160°C, [α]_{D} = +56,2° (EtOH)
1b. (-)-6-β-[exo-6-p-Fluor-phenyl-3-azabicyclo[3.2.0]heptan-3-yl]-ethyl-7-methyl-5H-thiazolo[3,2-a]pyrimidin-5-on Maleinat, Schmp. 147-149°C, [α]_{D} =-52,8° (EtOH)
2. 6-β-[exo-6-Phenyl-3-azabicyclo[3.2.0]heptan-3-yl]-ethyl-7-methyl-5H-thiazolo[3,2-a]pyrimidin-5-on, Schmp. 167-168°C (Maleinat),
3. 6-β-[exo-6,7-Diphenyl-3-azabicyclo[3.2.0]heptan-3-yl]-ethyl-7-methyl-5H-thiazolo[3,2-a]pyrimidin-5-on, Schmp. 154-156°C,
4. 6-β-[exo-6,7-bis-(p-Fluor-phenyl)-3-azabicyclo[3.2.0]heptan-3-yl]-ethyl-7-methyl-5H-thiazolo[3,2-a]pyrimidin-5-on,
5. 6-β-[exo-6-m-Chlor-phenyl-3-azabicyclo[3.2.0]heptan-3-yl]ethyl-7-methyl-5H-thiazolo[3,2-a]pyrimidin-5-on, Schmp. 162-164°C (Maleinat),
6. 6-β-[exo-6-m-Methoxy-phenyl-3-azabicyclo[3.2.0]-heptan-3-yl]ethyl-7-methyl-5H-thiazolo[3,2-a]pyrimidin-5-on, Schmp. 149-152°C (Maleinat)
7. 6-β-[exo-6-m-Hydroxy-phenyl-3-azabicyclo[3.2.0]heptan-3-yl]-ethyl-7-methyl-5H-thiazolo[3,2-a]pyrimidin-5-on (siehe auch Beispiel 49), Schmp. 76-78°C
8. 6-β-(2-[exo-6-p-Amino-phenyl-3-azabicyclo[3.2.0]heptan-3-yl]-ethyl-7-methyl-5H-thiazolo[3,2-a]pyrimidin-5-on (siehe auch Beispiel 50),
9. 6-β-[exo-6-p-Chlor-phenyl-3-azabicyclo[3.2.0]heptan-3-yl]-ethyl-7-methyl-5H-thiazolo[3,2-a]pyrimidin-5-on, Schmp. 155-157°C (Maleinat)
10. 6-β-[exo-6-p-Methoxy-phenyl-3-azabicyclo[3.2.0]heptan-3-yl]-ethyl-7-methyl-5H-thiazolo[3,2-a]pyrimidin-5-on, Schmp. 168-170°C (Maleinat)
11. 6-β-[exo-6-p-Nitro-phenyl-3-azabicyclo[3.2.0]heptan-3-yl]-ethyl-7-methyl-5H-thiazolo[3,2-a]pyrimidin-5-on,
12. 6-β-[exo-6-m-Fluor-phenyl-3-azabicyclo[3.2.0]heptan-3-yl]-ethyl-7-methyl-5H-thiazolo[3,2-a]pyrimidin-5-on,
13. 6-β-[exo-6-p-Hydroxy-phenyl-3-azabicyclo[3.2.0]heptan-3-yl]-ethyl-7-methyl-5H-thiazolo[3,2-a]pyrimidin-5-on (siehe auch Beispiel 49),
14. 6-β-[exo-6-p-Trifluormethyl-phenyl-3-azabicyclo[3.2.0]heptan-3-yl]-ethyl-7-methyl-5H-thiazolo[3,2-a]pyrimidin-5-on, Schmp. 187-189°C (Maleinat)
15. 6-β-[exo-6-(p-t-Butyl-phenyl)-3-azabicyclo[3.2.0]heptan-3-yl]-ethyl-7-methyl-5H-thiazolo[3,2-a]pyrimidin-5-on, Zersetzungspunkt 207-209°C (Maleinat)
16. 6-β-[endo-6-(p-t-Butyl-phenyl)-3-azabicyclo[3.2.0]heptan-3-yl]-ethyl-7-methyl-5H-thiazolo[3,2-a]pyrimidin-5-on, Schmp. 131-133°C
17. 6-β-[exo-6-(3,4-Dichlor-phenyl)-3-azabicyclo[3.2.0]heptan-3-yl]-ethyl-7-methyl-5H-thiazolo[3,2-a]pyrimidin-5-on,
18. 6-β-[exo-6-(3,4-Dimethoxy-phenyl)-3-azabicyclo[3.2.0]heptan-3-yl]-ethyl-7-methyl-5H-thiazolo[3,2-a]pyrimidin-5-on, Zersetzungspunkt 210-212°C (Dihydrochlorid)
19. 6-β-[exo-6-p-Cyano-phenyl-3-azabicyclo[3.2.0]heptan-3-yl]-ethyl-7-methyl-5H-thiazolo[3,2-a]pyrimidin-5-on, Schmp. 130-132°C
20. 6-β-[exo-6-(3,4-Dihydroxy-phenyl)-3-azabicyclo[3.2.0]heptan-3-yl]-ethyl-7-methyl-5H-thiazolo[3,2-a]pyrimidin-5-on (siehe auch Beispiel 49),
21. 6-β-[exo-6-(o-Fluor-phenyl-)-3-azabicyclo[3.2.0]heptan-3-yl]-ethyl-7-methyl-5H-thiazolo[3,2-a]pyrimidin-5-on, Schmp. 164-165°C (Maleinat)
22. 6-β-[exo-6-Thien-3-yl-3-azabicyclo[3.2.0]heptan-3-yl]ethyl-7-methyl-5H-thiazolo[3,2-a]pyrimidin-5-on,
23. 6-β-[exo-6-(5-Chlor-thien-2-yl)-3-azabicyclo[3.2.0]heptan-3-yl]-ethyl-7-methyl-5H-thiazolo[3,2-a]pyrimidin-5-on,
24. 6-β-[exo-6-pyrrol-2-yl-3-azabicyclo[3.2.0]heptan-3-yl]ethyl-7-methyl-5H-thiazolo[3,2-a]pyrimidin-5-on,
25. 6-β-[exo-6-pyrid-4-yl-3-azabicyclo[3.2.0]heptan-3-yl]ethyl-7-methyl-5H-thiazolo[3,2-a]pyrimidin-5-on,
26. 6-β-[exo-6-pyrid-3-yl-3-azabicyclo[3.2.0]heptan-3-yl]ethyl-7-methyl-5H-thiazolo[3,2-a]pyrimidin-5-on,
27. 6-β-[exo-6-p-Fluor-phenyl-3-azabicyclo[3.2.0]heptan-3-yl]ethyl-7-methyl-2,3-dihydro-5H-thiazolo[3,2-a]pyrimidin-5-on, Schmp. 253-255°C,
28. 6-β-[exo-6-m-Chlor-phenyl-3-azabicyclo[3.2.0]heptan-3-yl]ethyl-7-methyl-2,3-dihydro-5H-thiazolo[3,2-a]pyrimidin-5-on,
29. 6-β-[exo-6-p-Fluor-phenyl-3-azabicyclo[3.2.0]heptan-3-yl]ethyl-2,3,7-trimethyl-5H-thiazolo[3,2-a]pyrimidin-5-on,
30. 6-β-[exo-6-p-Fluor-phenyl-3-azabicyclo[3.2.0]heptan-3-yl]ethyl-5H-thiazolo[3,2-a]pyrimidin-5-on, Schmp. 164-166°C (Dihydrochlorid x H₂O)
31. 6-β-[exo-6-Phenyl-3-azabicyclo[3.2.0]heptan-3-yl]ethyl-5H-thiazolo[3,2-a]pyrimidin-5-on.

### Beispiel 32

7-β-[exo-6-p-Fluor-phenyl-3-azabicyclo[3.2.0]heptan-3-yl]-ethyl-8-methyl-3,4-dihydro-2H,6H-pyrimido[2,1-b] [1,3]thiazin-6-on Dihydrochlorid 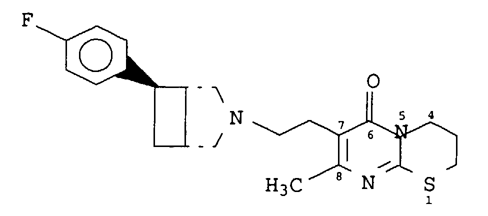 2,5 g (13,1 mM) exo-6-p-Fluor-phenyl-3-azabicyclo[3.2.0]heptan in 40 ml Xylol wurden mit 3,3 g (13,6 mM) 7-Chlorethyl-8-methyl-3,4-dihydro-2H,6H-pyrimido[2,1-b] [1,3] thiazin-6-on sowie mit 5,0 g (36 mM) fein pulverisiertem Kaliumcarbonat und 0,5 g Kaliumjodid versetzt und unter gutem Rühren 12 h unter Rückfluß gekocht.

Nach dem Abkühlen engte man am Rotationsverdampfer ein und verteilte den Rückstand zwischen Methylenchlorid und Wasser.

Die wäßrige Phase wurde zweimal mit Methylenchlorid nachextrahiert und darauf die organische Phase nach dem Trocknen mit Natriumsulfat eingeengt. Das Rohprodukt (5,6 g) reinigte man durch Säulenchromatographie (Kieselgel, Laufmittel Methylenchlorid/Methanol 93/7).

Die freie Base nahm man in 200 ml Ether auf, filtrierte die unlöslichen Flocken ab und versetzte die Etherlösung mit überschüssiger etherischer Salzsäure. Anschließend saugte man die Festkörper unter Stickstoff in der Kälte ab, wusch das Hydrochlorid mit reichlich Ether nach und trocknete das Salz auf der Nutsche unter Stickstoff. Man isolierte 3,2 g (52 %) Produkt x 2 HCl, Schmp. 120 bis 121°C.

Analog lassen sich herstellen:
33. 7-β-[exo-6-Phenyl-3-azabicyclo[3.2.0]heptan-3-yl]-ethyl-8-methyl-3,4-dihydro-2H,6H-pyrimido[2,1-b] [1,3] thiazin-6-on,
34. 7-β-[exo-6-m-Chlor-phenyl-3-azabicyclo[3.2.0]heptan-3-yl]ethyl-8-methyl-3,4-dihydro-2H,6H-pyrimido[2,1-b] [1,3] thiazin-6-on,
35. 7-β-[exo-6-p-Chlor-phenyl-3-azabicyclo[3.2.0]heptan-3-yl]ethyl-8-methyl-3,4-dihydro-2H,6H-pyrimido[2,1-b] [1,3] thiazin-6-on,
36. 7-β-[exo-6-p-Methoxy-phenyl-3-azabicyclo[3.2.0]heptan-3-yl]ethyl-8-methyl-3,4-dihydro-2H,6H-pyrimido[2,1-b] [1,3] thiazin-6-on,
37. 7-β-[exo-6-m-Hydroxy-phenyl-3-azabicyclo[3.2.0]heptan-3-yl]-ethyl-8-methyl-3,4-dihydro-2H,6H-pyrimido[2,1-b] [1,3] thiazin-6-on (siehe auch Beispiel 48),
38. 3-β-[exo-6-p-Fluor-phenyl-3-azabicyclo[3.2.0]heptan-3-yl]ethyl-2,4-(lH,3H)-chinazolindion, Schmp. 158-160°C,
38a. (+)-3-β-[exo-6-p-Fluor-phenyl-3-azabicyclo[3.2.0]heptan-3-yl]-ethyl-2,4-(1H,3H)-chinazolindion, Schmp. 160-162°C, [α]_{D} = + 88,6° (CH₂Cl₂)
38b. (-)-3-β-[exo-6-p-Fluor-phenyl-3-azabicyclo[3.2.0]heptan-3-yl]-ethyl-2,4-(1H,3H)-chinazolindion, Schmp. 161-162°C, [α]_{D} = - 87,5° (CH₂Cl₂)
39. 3-β-[exo-6-Phenyl-3-azabicyclo[3.2.0]heptan-3-yl]-ethyl-2,4-(1H,3H)-chinazolindion, Schmp. 144-146°C,
40. 3-β-[exo-6-m-Fluor-phenyl-3-azabicyclo[3.2.0]heptan-3-yl]ethyl-2,4-(1H,3H)-chinazolindion,
41. 3-β-[exo-6-p-Cyano-phenyl-3-azabicyclo[3.2.0]heptan-3-yl]ethyl-2,4-(lH,3H)-chinazolindion, Schmp. 230-232°C,
42. 3-β-[exo-6-m-Chlor-phenyl-3-azabicyclo[3.2.0]heptan-3-yl]ethyl-2,4-(1H,3H)-chinazolindion, Schmp. 183-185°C,
43. 3-β-[exo-6-p-Hydroxy-phenyl-3-azabicyclo[3.2.0]heptan-3-yl]-ethyl-2,4-(1H,3H)-chinazolindion, Schmp. 220-223°C (siehe auch Beispiel 48),
44. 3-β-[exo-6-p-Chlor-phenyl-3-azabicyclo[3.2.0]heptan-3-yl]ethyl-2,4-(lH,3H)-chinazolindion, Schmp. 187-189°C
45. 3-β-[exo-6-m-Methoxy-phenyl-3-azabicyclo[3.2.0]heptan-3-yl]ethyl-2,4-(lH,3H)-chinazolindion, Schmp. 113-115°C,
46. 3-β-[exo-6-p-Nitro-phenyl-3-azabicyclo[3.2.0]heptan-3-yl]ethyl-2,4-(lH,3H)-chinazolindion, Schmp. 209-211°C,
47. 3-β-[endo-6-p-Nitro-phenyl-3-azabicyclo[3.2.0]heptan-3-yl]ethyl-2,4-(1H,3H)-chinazolindion, Schmp. 212-214°C,

### Beispiel 48

3-β-[exo-6-m-Hydroxy-phenyl-3-azabicyclo[3.2.0]heptan-3-yl]ethyl-2,4-(1H,3H)-chinazolindion 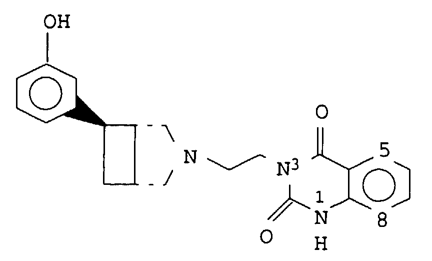 Zu 4,6 g (11,8 mM) 3-β-[exo-6-m-Methoxy-phenyl-3-azabicyclo[3.2.0]heptan-3-yl]-ethyl-2,4-(lH,3H)-chinazolindion in 120 ml Methylenchlorid wurden 28,5 ml (28,5 mM) Bortribromid (1 M Lösung in Methylenchlorid) bei Raumtemperatur getropft und das Gemisch über Nacht gerührt. Nach Kühlen wurden 100 ml 2 N Natronlauge hinzugegeben, die organische Phase abgetrennt und die wäßrige Phase mit Methylenchlorid nachextrahiert. Nach Trocknen und Einengen erhielt man 4,7 g Rohprodukt, das durch Säulenchromatographie (Kieselgel, Laufmittel Methylenchlorid/Methanol 96/4) gereinigt wurde. Ausbeute: 2,8 g (61 %), Schmp. 149-151°C (Hydrochlorid).

### Beispiel 49

### 3-β-[exo-6-p-Amino-phenyl-3-azabicyclo[3.2.0]heptan-3-yl)ethyl-2,4-(1H,3H)-chinazolindion

16,3 g (40,1 mM) 3-β-[exo-6-p-Nitro-phenyl-3-azabicyclo[3.2.0]heptan-3-yl]-ethyl-2,4-(lH,3H)-chinazolindion wurden in 300 ml Eisessig gelöst, mit 1,7 g Palladium auf Kohle (10 %) versetzt und 4 h bei Raumtemperatur und Normaldruck hydriert. Nach Absaugen des Katalysators engte man die Mutterlauge ein, nahm den Rückstand in 400 ml Wasser auf, stellte unter Rühren mit konzentriertem Ammoniak alkalisch und saugte die ausgefallenen Festkörper unter Nachwaschen mit Wasser ab. Das Rohprodukt (15,3 g) reinigte man durch Säulenchromatographie (Kieselgel, Laufmittel Methylenchlorid/Methanol 95/5). Ausbeute: 12,4 g (76 %) mit Schmp. 196-198°C.

### Beispiel 50

### 3-β-[exo-6-p-Jod-phenyl-3-azabicyclo[3.2.0]heptan-3-yl)-ethyl-2,4-(1H,3H)-chinazolindion

5,6 g (14,9 mM) 3-β-[exo-6-p-Amino-phenyl-3-azabicyclo[3.2.0]heptan-3-yl)-ethyl-2,4-(1H,3H)-chinazolindion wurden in 100 ml halbkonzentrierter Salzsäure gelöst. Bei 0-5°C tropfte man eine Lösung von 1,05 g (15,0 mM) Natriumnitrit in 6 ml Wasser hinzu und ließ den Ansatz noch 20 min bei der gleichen Temperatur nachrühren. Anschließend fügte man eine Lösung von 2,5 g (15,0 mM) Kaliumjodid in 12 ml Wasser hinzu, entfernte das Eisbad und heizte die Mischung langsam unter gutem Rühren bis auf 85-90°C auf. Nach 40 min ließ man abkühlen, setzte Eis/Wasser hinzu, stellte mit konzentriertem Ammoniak alkalisch, fügte 300 ml Methylenchlorid hinzu und rührte intensiv nach. Nach Phasentrennung extrahierte man die wäßrige Phase mit Methylenchlorid nach, trocknete die vereinten organischen Phasen und engte ein. Das Rohprodukt (6,0 g) reinigte man durch Säulenchromatographie (Kieselgel, Laufmittel Methylenchlorid/Methanol 95/5). Ausbeute: 3,2 g (57 %), Schmp. 162-164°C.
51. 3-β-[exo-6-p-Trifluormethyl-phenyl-3-azabicyclo[3.2.0]heptan-3-yl]-ethyl-2,4-(1H,3H)-chinazolindion, Schmp. 190-192°C
52. 3-β-[exo-6-(3,4-Dichlor-phenyl)-3-azabicyclo[3.2.0]heptan-3-yl]-ethyl-2,4-(1H,3H)-chinazolindion,
53. 3-β-[exo-6-(3,4-Dihydroxy-phenyl)-3-azabicyclo[3.2.0]heptan-3-yl]-ethyl-2,4-(1H,3H)-chinazolindion (analog Beispiel 48),
54. 3-β-[exo-6-(3,5-Dichlor-phenyl)-3-azabicyclo13.2.0]heptan-3-yl]-ethyl-2,4-(1H,3H)-chinazolindion, Schmp. 189-192°C,
55. 3-β-[exo-6-o-Fluor-phenyl-3-azabicyclo[3.2.0]heptan-3-yl]ethyl-2,4-(1H,3H)-chinazolindion,
56. 3-β-[exo-6-p-Fluor-phenyl-3-azabicyclo[3.2.0]heptan-3-yl]ethyl-8-methyl-2,4-(1H,3H)-chinazolindion, Schmp. 170-173°C,
57. 3-β-[exo-6-p-Fluor-phenyl-3-azabicyclo[3.2.0]heptan-3-yl]ethyl-7-chlor-2,4-(1H,3H)-chinazolindion, Schmp. 214-216°C,
58. 3-β-[exo-6-p-Fluor-phenyl-3-azabicyclo[3.2.0]heptan-3-yl]ethyl-7-fluor-2,4-(1H,3H)-chinazolindion,
59. 3-β-[exo-6-p-Fluor-phenyl-3-azabicyclo[3.2.0]heptan-3-yl]ethyl-5-chlor-2,4-(lH,3H)-chinazolindion, Schmp.
60. 3-β-[exo-6-p-Fluor-phenyl-3-azabicyclo[3.2.0]heptan-3-yl]ethyl-6-fluor-2,4-(1H,3H)-chinazolindion, Schmp. 186-188°C,
61. 3-β-[exo-6-p-Fluor-phenyl-3-azabicyclo[3.2.0]heptan-3-yl]ethyl-6-methyl-2,4-(1H,3H)-chinazolindion, Schmp. 166-168°C,
62. 3-β-[exo-6-p-Fluor-phenyl-3-azabicyclo[3.2.0]heptan-3-yl]ethyl-8-methoxy-2,4-(1H,3H)-chinazolindion,
63. 3-β-[exo-6-p-Fluor-phenyl-3-azabicyclo[3.2.0]heptan-3-yl]ethyl-6-trifluormethyl-2,4-(1H,3H)-chinazolindion,
64. 3-β-[exo-6-p-Fluor-phenyl-3-azabicyclo[3.2.0]heptan-3-yl]ethyl-7-nitro-2,4-(1H,3H)-chinazolindion,
65. 3-β-[exo-6-p-Fluor-phenyl-3-azabicyclo[3.2.0]heptan-3-yl]ethyl-6-nitro-2,4-(lH,3H)-chinazolindion,
66. 3-β-[exo-6-p-Fluor-phenyl-3-azabicyclo[3.2.0]heptan-3-yl]ethyl-7-amino-2,4-(lH,3H)-chinazolindion,
67. 3-β-[exo-6-p-Fluor-phenyl-3-azabicyclo[3.2.0]heptan-3-yl]ethyl-6-amino-2,4-(lH,3H)-chinazolindion,
68. 3-β-[exo-6-p-Fluor-phenyl-3-azabicyclo[3.2.0]heptan-3-yl]ethyl-6-hydroxy-2,4-(1H,3H)-chinazolindion,
69. 3-β-[exo-6-p-Fluor-phenyl-3-azabicyclo[3.2.0]heptan-3-yl]ethyl-5-chlor-2,4-(lH,3H)-chinazolindion, Schmp. 194-196°C (Maleinat),
70. 3-β-[exo-6-p-Fluor-phenyl-3-azabicyclo[3.2.0]heptan-3-yl]ethyl-6,7-dimethoxy-2,4-(1H,3H)-chinazolindion, Schmp. 203-205°C,
71. 3-β-[exo-6-p-Fluor-phenyl-3-azabicyclo[3.2.0]heptan-3-yl]ethyl-6,8-dichlor-2,4-(1H,3H)-chinazolindion,
72. 3-β-[exo-6-p-Fluor-phenyl-3-azabicyclo[3.2.0]heptan-3-yl]ethyl-6,7,8-trimethoxy-2,4-(1H,3H)-chinazolindion,
73. 3-β-[exo-6-p-Fluor-phenyl-3-azabicyclo[3.2.0]heptan-3-yl]ethyl-1-methyl-2,4-(1H,3H)-chinazolindion, Schmp. 89-90°C,
74. 3-β-[exo-6-p-Fluor-phenyl-3-azabicyclo[3.2.0]heptan-3-yl]ethyl-1-ethyl-2,4-(1H,3H)-chinazolindion, Schmp. 92-95°C (Hydrochlorid),
75. 3-β-[exo-6-p-Fluor-phenyl-3-azabicyclo[3.2.0]heptan-3-yl]ethyl-1-allyl-2,4-(1H,3H)-chinazolindion,
76. 3-β-[exo-6-p-Fluor-phenyl-3-azabicyclo[3.2.0]heptan-3-yl]ethyl-1-benzyl-2,4-(1H,3H)-chinazolindion, Schmp. 133-135°C,
77. 3-y-[exo-6-p-Fluor-phenyl-3-azabicyclo[3.2.0]heptan-3-yl]propyl-2,4-(1H,3H)-chinazolindion, Schmp. 75-77°C,
78. 3-δ-[exo-6-p-Fluor-phenyl-3-azabicyclo[3.2.0]heptan-3-yl]butyl-2,4-(1H,3H)-chinazolindion,
79. 3-β-[exo-6-(2-Thienyl)-3-azabicyclo[3.2.0]heptan-3-yl]ethyl-2,4-(1H,3H)-chinazolindion, Schmp. 171-173°C,
80. 3-β-[exo-6-(5-Chlor-2-thienyl)-3-azabicyclo[3.2.0]heptan-3-yl]-ethyl-2,4-(1H,3H)-chinazolindion, Zersetzungspunkt ab 176°C,
81. 3-β-[exo-6-(3-Thienyl)-3-azabicyclo[3.2.0]heptan-3-yl]ethyl-2,4-(1H,3H)-chinazolindion, Schmp. 158-159°C,
82. 3-β-[exo-6-(3-Pyridyl)-3-azabicyclo[3.2.0]heptan-3-yl]ethyl-2,4-(1H,3H)-chinazolindion, Zersetzungspunkt ab 84°C,
83. 3-β-[exo-6-p-Fluor-phenyl-3-azabicyclo[3.2.0]heptan-3-yl]ethyl-1H-thieno[3,2-d]pyrimidin-2,4-dion, Schmp. 230-232°C,
84. 3-β-[exo-6-p-Fluor-phenyl-3-azabicyclo[3.2.0]heptan-3-yl]ethyl-lH-thieno[2,3-d]pyrimidin-2,4-dion,
85. 3-β-[exo-6-p-Fluor-phenyl-3-azabicyclo[3.2.0]heptan-3-yl]ethyl-1H-thieno[3,4-d]pyrimidin-2,4-dion,
86. 3-β-[exo-6-p-Fluor-phenyl-3-azabicyclo[3.2.0]heptan-3-yl]ethyl-1-methyl-1H,3H-pyrido[2,3-d]pyrimidin-2,4-dion,
87. 3-β-[exo-6-p-Fluor-phenyl-3-azabicyclo[3.2.0]heptan-3-yl]ethyl-2-methyl-3H-chinazolin-4-on, Zersetzungspunkt ab 225°C (Hydrochlorid),
88. 3-β-[exo-6-p-Fluor-phenyl-3-azabicyclo[3.2.0]heptan-3-yl]ethyl-2-methoxy-3H-chinazolin-4-on

### Beispiel 89

### 3-β-[exo-6-p-Fluor-phenyl-3-azabicyclo[3.2.0]heptan-3-yl]-ethyl-2-methyl-4H-pyrido[1,2-a]pyrimidin-4-on Dihydrochlorid

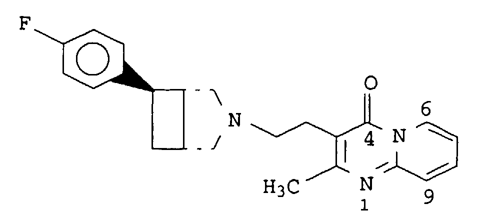

3,0 g (15,7 mM) exo-6-p-Fluor-phenyl-3-azabicyclo[3.2.0]heptan in 60 ml Xylol wurden mit 3,8 g (17 mM) 3-(2-Chlor-ethyl)-2-methyl-4H-pyrido[1,2-a]pyrimidin-4-on sowie mit 4,2 g (30 mM) fein pulverisiertem Kaliumcarbonat und 0,5 g Kaliumjodid versetzt und unter gutem Rühren 11 h unter Rückfluß gekocht. Nach dem Abkühlen engte man am Rotationsverdampfer ein und verteilte den Rückstand zwischen Methylenchlorid und Wasser. Die wäßrige Phase wurde zweimal mit Methylenchlorid nachextrahiert und darauf die organische Phase nach dem Trocknen mit Natriumsulfat eingeengt. Das Rohprodukt (7,8 g) reinigte man durch Säulenchromatographie (Kieselgel, Laufmittel Methylenchlorid/Methanol 94/4). Die freie Base (3,4 g) nahm man in 200 ml Ether auf, filtrierte die unlöslichen Flocken ab und versetzte die Etherlösung mit überschüssiger etherischer Salzsäure. Anschließend saugte man die Festkörper unter Stickstoff in der Kälte ab, wusch das Hydrochlorid mit reichlich Ether nach und trocknete das Salz auf der Nutsche unter Stickstoff. Man isolierte 3,8 g (54 %) Produkt x 2 HCl, Schmp. > 250°C.

Analog lassen sich herstellen:
90. 3-β-[exo-6-Phenyl-3-azabicyclo[3.2.0]heptan-3-yl]-ethyl-2-methyl-4H-pyrido[1,2-a]pyrimidin-4-on,
91. 3-β-[exo-6-m-Chlor-phenyl-3-azabicyclo[3.2.0]heptan-3-yl]ethyl-2-methyl-4H-pyrido[1,2-a]pyrimidin-4-on,
92. 3-β-[exo-6-m-Methoxy-phenyl-3-azabicyclo[3.2.0]heptan-3-yl]-ethyl-2-methyl-4H-pyrido[1,2-a]pyrimidin-4-on,
93. 3-β-[exo-6-p-Fluor-phenyl-3-azabicyclo[3.2.0]heptan-3-yl]-ethyl-2,6-dimethyl-4H-pyrido[1,2-a]pyrimidin-4-on, Schmp. 59-61°C (Dihydrochlorid),
94. 3-β-[exo-6-p-Fluor-phenyl-3-azabicyclo[3.2.0]heptan-3-yl]-ethyl-2,7-dimethyl-4H-pyrido[1,2-a]pyrimidin-4-on, Schmp. 247-249°C (Dihydrochlorid),
95. 3-β-[exo-6-p-Fluor-phenyl-3-azabicyclo[3.2.0]heptan-3-yl]-ethyl-2,8-dimethyl-4H-pyrido[1,2-a]pyrimidin-4-on, Schmp. >250°C (Dihydrochlorid),
96. 3-β-[exo-6-p-Fluor-phenyl-3-azabicyclo[3.2.0]heptan-3-yl]-ethyl-2,9-dimethyl-4H-pyrido[1,2-a]pyrimidin-4-on, Zersetzungspunkt >208°C (Dihydrochlorid),
97. 3-β-[exo-6-p-Fluor-phenyl-3-azabicyclo[3.2.0]heptan-3-yl]-ethyl-2,6,8-trimethyl-4H-pyrido[1,2-a]pyrimidin-4-on, Schmp. >260°C (Dihydrochlorid),
98. 3-β[exo-6-m-Chlor-phenyl-3-azabicyclo[3.2.0]heptan-3-yl]-ethyl-2,7-dimethyl-4H-pyrido[1,2-a]pyrimidin-4-on, Schmp. >250°C (Dihydrochlorid),
99. 3-β[exo-6-o-Fluor-phenyl-3-azabicyclo[3.2.0]heptan-3-yl]-ethyl-2,7-dimethyl-4H-pyrido[1,2-a]pyrimidin-4-on, Schmp. 262-264°C (Dihydrochlorid),
100. 3-β-[exo-6-Phenyl-3-azabicyclo [3.2.0] heptan-3-yl] -ethyl-2,8-dimethyl-4H-pyrido[1,2-a]pyrimidin-4-on, Schmp. >250°C (Dihydrochlorid),
101. 3-β-[exo-6-m-Chlor-phenyl-3-azabicyclo[3.2.0]heptan-3-yl]-ethyl-2,9-dimethyl-4H-pyrido[1,2-a]pyrimidin-4-on, Zersp. >213°C (Dihydrochlorid),
102. 3-β-[exo-6-(5-Chlor-thien-2-yl)-3-azabicyclo[3.2.0]heptan-3-yl]-ethyl-2-methyl-4H-pyrido[1,2-a]pyrimidin-4-on,
103. 3-β-[exo-6-pyrid-4-yl-3-azabicyclo[3.2.0]heptan-3-yl]ethyl-2-methyl-4H-pyrido[1,2-a]pyrimidin-4-on,
104. 3-β-[exo-6-p-Fluor-phenyl-3-azabicyclo[3.2.0]heptan-3-yl]ethyl-6,7,8,9-tetrahydro-2-methyl-4H-pyrido[1,2-a]pyrimidin-4-on, Schmp. 151-153° (Maleinat),
105. 3-β-[exo-6-p-Chlor-phenyl-3-azabicyclo[3.2.0]heptan-3-yl]ethyl-6,7,8,9-tetrahydro-2-methyl-4H-pyrido[1,2-a]pyrimidin-4-on,
106. 3-β-[exo-6-p-Methoxy-phenyl-3-azabicyclo[3.2.0]heptan-3-yl]ethyl-6,7,8,9-tetrahydro-2-methyl-4H-pyrido[1,2-a]pyrimidin-4-on,
107. 3-β-[exo-6-p-Fluor-phenyl-3-azabicyclo[3.2.0]heptan-3-yl]ethyl-2-methyl-4H-pyrimido[1,2-a]pyrimidin-4-on,
108. 3-β-[exo-6-p-Fluor-phenyl-3-azabicyclo [3.2.0]heptan-3-yl]ethyl-2-methyl-7-chlor-4H-pyrimido[1,2-a]pyrimidin-4-on,
109. 6-β-[exo-6-p-Fluor-phenyl-3-azabicyclo[3.2.0]heptan-3-yl]ethyl-7-methyl-1H,5H-imidazo[1,2-a]pyrimidin-5-on,
110. 3-β-[exo-6-p-Fluor-phenyl-3-azabicyclo[3.2.0]heptan-3-yl]ethyl-2-methyl-7,8-dihydro-4H,6H-pyrrolo[1,2-a]pyrimidin-4-on,
111. 2-[exo-6-p-Fluor-phenyl-3-azabicyclo[3.2.0]heptan-3-yl]methyl-benzimidazol, Schmp. 166-168°C,
112. 1-β-[exo-6-p-Fluor-phenyl-3-azabicyclo[3.2.0]heptan-3-yl]ethyl-benzimidazol, Schmp. 94-96°C (Hydrochlorid),
113. 1-β-[exo-6-p-Fluor-phenyl-3-azabicyclo[3.2.0]heptan-3-yl]ethyl-2,3-dihydro-benzimidazol-2-on,
114. 2-[exo-6-m-Methoxy-phenyl-3-azabicyclo[3.2.0]heptan-3-yl]methyl-benzimidazol,
115. 3-β-[exo-6-p-Fluor-phenyl-3-azabicyclo[3.2.0]heptan-3-yl]ethyl-indol, Schmp. 193-195°C (Hydrochlorid),
116. 3-β-(2-[exo-6-m-Chlor-phenyl-3-azabicyclo[3.2.0]heptan-3-yl]-ethyl-indol,
117. 3-β-[exo-6-Phenyl-3-azabicyclo[3.2.0]heptan-3-yl]-ethylindol, Schmp. 104-105°C (Hydrochlorid),
118. 3-β-[exo-6-m-Methoxy-phenyl-3-azabicyclo[3.2.0]heptan-3-yl]ethyl-indol,
119. 2-[exo-6-p-Fluor-phenyl-3-azabicyclo[3.2.0]heptan-3-yl]methyl-3,4-dihydro-chinazolin-4-on, Schmp. 152 bis 154°C,
120. 2-[exo-6-p-Fluor-phenyl-3-azabicyclo[3.2.0]heptan-3-yl]methyl-3,4-dihydro-chinazolin-4-thion,
121. 2-[exo-6-Phenyl-3-azabicyclo[3.2.0]heptan-3-yl]-methyl-3,4-dihydro-chinazolin-4-on, Schmp. 147-149°C,
122. 2-[exo-6-m-Chlor-phenyl-3-azabicyclo[3.2.0]heptan-3-yl]methyl-3,4-dihydro-chinazolin-4-on,
123. 2-[exo-6-o-Methoxy-phenyl-3-azabicyclo[3.2.0]heptan-3-yl]methyl-3,4-dihydro-chinazolin-4-on,
124. 2-[exo-6-p-Fluor-phenyl-3-azabicyclo[3.2.0]heptan-3-yl]methyl-4-hydroxy-6-methyl-pyrimidin, Schmp. 174-175°C,
125. 6-[exo-6-p-Fluor-phenyl-3-azabicyclo[3.2.0]heptan-3-yl]methyl-2-methyl-4-hydroxy-pyrimidin, Schmp. 147-149°C (Dihydrochlorid),
126. 6-[exo-6-p-Fluor-phenyl-3-azabicyclo[3.2.0]heptan-3-yl]methyl-uracil, Schmp. 201-203°C,
127. 6-[exo-6-Phenyl-3-azabicyclo[3.2.0]heptan-3-yl]-methyl-uracil, Schmp. 183-184°C,
128. 6-[exo-6-p-Fluor-phenyl-3-azabicyclo[3.2.0]heptan-3-yl]-1,2,3,4-tetrahydro-1,3-dimethyl-2,4-dioxo-pyrimidin, Schmp. 108-110°C (Hydrochlorid),
129. 5-β-[exo-6-p-Fluor-phenyl-3-azabicyclo[3.2.0]heptan-3-yl]-ethyl-1,2,3,4-tetrahydro-6-methyl-2,4-dioxo-pyrimidin, Schmp. 197-199°C,
130. 5-β-[exo-6-p-Fluor-phenyl-3-azabicyclo[3.2.0]heptan-3-yl]ethyl-1,2,3,4-tetrahydro-1,6-dimethyl-2,4-dioxo-pyrimidin, Schmp. 186-188°C,
131. 5-β-[exo-6-p-Fluor-phenyl-3-azabicyclo[3.2.01heptan-3-yl]ethyl-1,2,3,4-tetrahydro-3,6-dimethyl-2,4-dioxo-pyrimidin,
132. 5-β-[exo-6-p-Fluor-phenyl-3-azabicyclo[3.2.0]heptan-3-yl]ethyl-1,2,3,4-tetrahydro-3,6-dimethyl-2,4-dithioxo-pyrimidin,
133. 5-β-[exo-6-p-Fluor-phenyl-3-azabicyclo [3.2.0] heptan-3-yl]ethyl-1,2,3,4-tetrahydro-1,3,6-trimethyl-2,4-dioxo-pyrimidin Schmp. 90-93°C (Hydrochlorid),
134. 5-β-[exo-6-p-Fluor-phenyl-3-azabicyclo[3.2.0]heptan-3-yl]ethyl-2-thiomethyl-6-methyl-4(3H)-pyrimidinon,
135. 5-β-[exo-6-p-Fluor-phenyl-3-azabicyclo[3.2.0]heptan-3-yl]ethyl-2-mercapto-6-methyl-4(3H)-pyrimidinon,
136. 5-β-[exo-6-p-Fluor-phenyl-3-azabicyclo[3.2.0]heptan-3-yl]ethyl-2-thiomethyl-3,6-dimethyl-4(3H)-pyrimidinon, Schmp. 132-135°C (Dihydrochlorid),
137. 5-β-[exo-6-p-Fluor-phenyl-3-azabicyclo[3.2.0]heptan-3-yl]ethyl-1,2,3,4-tetrahydro-6-amino-l-methyl-2,4-dioxopyrimidin,
138. 5-β-[exo-6-p-Fluor-phenyl-3-azabicyclo[3.2.0]heptan-3-yl]ethyl-2-amino-6-methyl-4(3H)-pyrimidinon,
139. 5-β-[exo-6-p-Fluor-phenyl-3-azabicyclo[3.2.0]heptan-3-yl]ethyl-2-amino-3,6-dimethyl-4(3H)-pyrimidinon, Schmp. 78-80°C,
140. 5-β-[exo-6-p-Fluor-phenyl-3-azabicyclo[3.2.0]heptan-3-yl]ethyl-2-methylamino-3,6-dimethyl-4(3H)-pyrimidinon, Schmp. 163-165°C (Dihydrochlorid),
141. 5-β-[exo-6-p-Fluor-phenyl-3-azabicyclo[3.2.0]heptan-3-yl]ethyl-2-dimethylamino-4(3H)-pyrimidinon,
142. 5-β-[exo-6-p-Fluor-phenyl-3-azabicyclo[3.2.0]heptan-3-yl]ethyl-2-methylamino-3-ethyl-6-methyl-4(3H)-pyrimidinon,
143. 5-β-[exo-6-p-Fluor-phenyl-3-azabicyclo[3.2.0]heptan-3-yl]ethyl-2-amino-3-ethyl-6-methyl-4(3H)-pyrimidinon, Schmp. 77-80°C (Hydrochlorid),
144. 5-β-[exo-6-p-Fluor-phenyl-3-azabicyclo[3.2.0]heptan-3-yl]ethyl-2-ethylamino-3,6-dimethyl-4(3H)-pyrimidinon,
145. 5-β-[exo-6-p-Fluor-phenyl-3-azabicyclo[3.2.0]heptan-3-yl]ethyl-2-n-propylamino-3,6-dimethyl-4(3H)-pyrimidinon,
146. 5-β-[exo-6-p-Chlor-phenyl-3-azabicyclo [3.2.0] heptan-3-yl]ethyl-2-methylamino-3,6-dimethyl-4(3H)-pyrimidinon, Zersetzungspunkt ab 144°C (Dihydrochlorid x 2 H₂O),
147. 5-β-[exo-6-m-Chlor-phenyl-3-azabicyclo[3.2.0]heptan-3-yl]ethyl-2-methylamino-3,6-dimethyl-4(3H)-pyrimidinon, Schmp. 147-149°C (Dihydrochlorid),
148. 5-β-[exo-6-o-Fluor-phenyl-3-azabicyclo [3.2.0] heptan-3-yl]ethyl-2-methylamino-3,6-dimethyl-4(3H)-pyrimidinon, Schmp. 173-175°C (Dihydrochlorid),
149. 5-β-[exo-6-p-Trifluormethyl-phenyl-3-azabicyclo[3.2.0]heptan-3-yl]-ethyl-2-methylamino-3,6-dimethyl-4(3H)-pyrimidinon,
150. 5-β-[exo-6-p-Cyano-phenyl-3-azabicyclo[3.2.0]heptan-3-yl]-ethyl-2-methylamino-3,6-dimethyl-4(3H)-pyrimidinon,
151. 5-β-[exo-6-m-Fluor-phenyl-3-azabicyclo [3.2.0] heptan-3-yl]-ethyl-2-methylamino-3,6-dimethyl-4(3H)-pyrimidinon,
152. 5-β-[exo-6-p-Fluor-phenyl-3-azabicyclo[3.2.0]heptan-3-yl]-ethyl-2-methylamino-4-methoxy-6-methyl-pyrimidin.

## Patentansprüche

1. N-substituierte 3-Azabicyclo[3.2.0]heptan-Derivate der Formel I 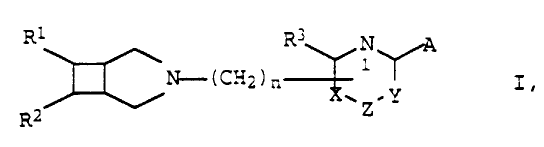 worin
R¹ eine gegebenenfalls durch Halogenatome, C₁-C₄-Alkyl, Trifluormethyl-, Hydroxy-, C₁-C₄-Alkoxy-, Amino-, Mono- methylamino-, Dimethylamino-, Cyano- oder Nitrogruppen mono- oder disubstituierte Phenyl-, Pyridyl-, Thienyl- oder Pyrrolgruppe bedeutet,
R² ein Wasserstoffatom oder eine gegebenenfalls durch Halogen, Methoxy, Hydroxy oder Amino substituierte Phenylgruppe ist,
n die Zahl 0, 1, 2, 3 oder 4 bedeutet,
R³ ein Wasserstoffatom, eine Hydroxy-, C₁-C₄-Alkyl- oder C₁-C₄-Alkoxygruppe ist, oder zusammen mit dem benachbarten Kohlenstoffatom eine C=O- oder C=S-Gruppe bedeutet,
X und Y Kohlenstoffatome, CH-, CH₂-, NH- oder C₁-C₄-Alkyl- N-Gruppen oder Stickstoffatome bedeuten,
Z eine direkte Bindung, eine CO-Gruppe, CS-Gruppe oder eine CH- bzw. CH₂-Gruppe, in denen ein Wasserstoffatom gegen eine Hydroxy-, Amino- oder C₁-C₄-Alkoxygruppe oder ein Halogenatom ausgetauscht sein kann, darstellt, und
A ein Wasserstoffatom, eine Hydroxy-, Amino-, Mercapto-, C₁-C₄-Alkylamino-, Di-C₁-C₄-alkylamino-, C₁-C₄-Alkylthio- oder C₁-C₄-Alkoxygruppe oder zusammen mit dem benachbarten Kohlenstoffatom eine C=O-Gruppe bedeutet, oder
A eine mit Y verknüpfte C₃-C₄-Alkylengruppe bedeutet, die eine oder zwei nicht kumulierte Doppelbindungen enthalten kann und in der eine CH- oder CH₂-Gruppe durch ein Stickstoff- oder Schwefelatom oder eine NH- oder N-CH₃-Gruppe ersetzt sein kann und wobei der Ring entweder durch ein Fluor- oder Chloratom oder eine Methyl-, Methoxy-, Nitro- oder Aminogruppe monosubstituiert oder im Fall eines Benzolrings dieser durch Fluor- oder Chloratome oder Methyl-, Trifluormethyl-, Nitro-, Hydroxy-, Methoxy-, Amino-, Monomethyl- oder Dimethylaminogruppen mono-, di- oder trisubstituiert sein kann,
und worin der Ring im rechten Teil der Formel I am Stickstoffatom Nr. 1 eine C₁-C₄-Alkyl-, Allyl- oder Benzylgruppe tragen und 1 bis 3 nicht kumulierte Doppelbindungen enthalten kann,
und deren Salze mit physiologisch verträglichen Säuren.

2. Verbindungen der Formel I gemäß Anspruch 1 zur Verwendung bei der Bekämpfung von Krankheiten.

## Claims

1. An N-substituted 3-azabicyclo[3.2.0]heptane derivative of the formula I 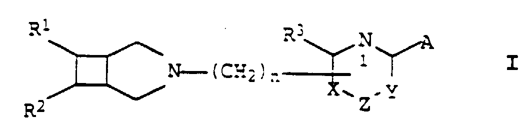 where
R¹ is phenyl, pyridyl, thienyl or pyrrolyl, each of which is unsubstituted or mono- or disubstituted by halogen, C₁-C₄-alkyl, trifluoromethyl, hydroxyl, C₁-C₄-alkoxy, amino, monomethylamino, dimethylamino, cyano or nitro,
R² is hydrogen or phenyl which is unsubstituted or substituted by halogen, methoxy, hydroxyl or amino,
n is 0, 1, 2, 3 or 4,
R³ is hydrogen, hydroxyl, C₁-C₄-alkyl or C₁-C₄-alkoxy, or together with the adjacent carbon atom is C=O or C=S,
X and Y are each C, CH, CH₂, NH or C₁-C₄-alkyl-N or N,
Z is a direct linkage, CO, CS or CH or CH₂ in which one hydrogen can be replaced by hydroxyl, amino, C₁-C₄-alkoxy or halogen, and
A is hydrogen, hydroxyl, amino, mercapto, C₁-C₄-alkylamino, di-C₁-C₄-alkylamino, C₁-C₄-alkylthio or C₁-C₄-alkoxy, or together with the adjacent carbon is C=O, or
A is C₃-C₄-alkylene which is attached to Y and which can contain one or two non-cumulative double bonds and in which one -CH or CH₂ can be replaced by N, S, NH or N-CH₃, and where the ring can be either monosubstituted by fluorine, chlorine, methyl, methoxy, nitro or amino or, in the case of a benzene ring, mono-, di- or trisubstituted by fluorine, chlorine, methyl, trifluoromethyl, nitro, hydroxyl, methoxy, amino, monomethylamino or dimethylamino,
and in which the ring on the right in formula I can have C₁-C₄-alkyl, allyl or benzyl attached to N-1 and can contain 1-3 non-cumulative double bonds, and the salts thereof with physiologically tolerated acids.

2. A compound of the formula I as claimed in claim 1 for use for controlling diseases.

## Revendications

1. Dérivés du 3-azabicyclo[3.2.0]heptane N-substitués de la formule I 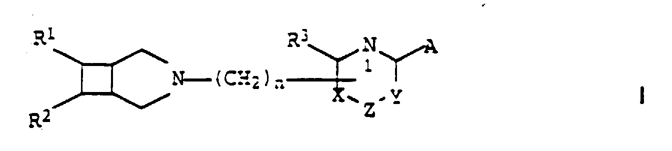 dans laquelle
R¹ représente un radical pyrrole, thiényle, pyridyle, ou phényle, éventuellement monosubstitué ou disubstitué par des atomes d'halogènes, des radicaux alkyle en C₁ à C₄, trifluorométhyle, hydroxyle, alcoxy en C₁ à C₄, amino, monométhylamino, diméthylamino, cyano ou nitro,
R² représente un atome d'hydrogène ou un radical phényle éventuellement substitué par des atomes d'halogènes, des radicaux méthoxy, hydroxyle ou amino,
n est égal à 0, 1, 2, 3, ou 4,
R³ représente un atome d'hydrogène, un radical hydroxyle, alkyle en C₁ à C₄, ou alcoxy en C₁ à C₄, ou bien forme ensemble avec l'atome de carbone voisin, un groupe C=O ou C=S,
X et Y représentent des atomes d'hydrogène, des radicaux CH-, CH₂-, NH-, ou alkyl(C₁ à C₄)-N, ou des atomes d'azote,
Z représente une liaison directe, un radical CO, un radical CS, ou un radical CH ou CH₂, dans lesquels un atome d'hydrogène peut être remplacé par un radical hydroxyle, amino ou alcoxy en C₁ à C₄, ou un atome d'halogène,
et
A représente un atome d'hydrogène, un radical hydroxyle, amino, mercapto, alkylamino en C₁ à C₄, dialkylamino en C₁ à C₄, alkylthio en C₁ à C₄, ou alcoxy en C₁ à C₄, ou forme, ensemble avec l'atome de carbone voisin, un groupe C=O, ou bien
A représente un groupe alkylène en C₃ à C₄ lié à Y, qui peut contenir une ou deux doubles liaisons non conjuguées et dans lequel un groupe CH ou CH₂ peut être remplacé par un atome d'azote ou de soufre ou un groupe NH ou N-CH₃ et où le noyau peut être monosubstitué par un atome de fluor ou de chlore, ou par un groupe méthyle, méthoxy, nitro ou amino, ou bien, dans le cas d'un noyau benzénique, celui-ci peut être monosubstitué, disubstitué ou trisubstitué par des atomes de fluor ou de chlore ou des radicaux méthyle, trifluorométhyle, nitro, hydroxyle, méthoxy, amino, monométhyle ou diméthylamino.
et où le noyau dans la partie droite de la formule I peut porter, sur l'atome d'azote n° 1, un radical alkyle en C₁ à C₄, allyle, ou benzyle et contenir une à trois doubles liaisons non conjuguées ainsi que leurs sels avec des acides physiologiquement acceptables.

2. Composés suivant la revendication 1, à utiliser pour la lutte contre des maladies.
